(19) **Europäisches Patentamt / European Patent Office / Office européen des brevets**

(11) **EP 4 080 191 B1**

(12) **EUROPEAN PATENT SPECIFICATION**

(45) Date of publication and mention of the grant of the patent:
**01.04.2026 Bulletin 2026/14**

(21) Application number: **20845705.1**

(22) Date of filing: **16.12.2020**

(51) International Patent Classification (IPC):
*G01N 15/10* (2024.01)    *G16B 40/00* (2019.01)
*G05B 13/02* (2006.01)    *G01N 15/14* (2024.01)
*G01N 15/1031* (2024.01)    *G01N 15/1429* (2024.01)
*G16H 10/60* (2018.01)    *G16H 50/70* (2018.01)

(52) Cooperative Patent Classification (CPC):
**G01N 15/1031; G01N 15/1459; G16H 10/60; G16H 50/70;** G01N 15/1429; G01N 2015/1006

(86) International application number:
**PCT/PT2020/050052**

(87) International publication number:
**WO 2021/125987 (24.06.2021 Gazette 2021/25)**

(54) **METHOD FOR DETECTION AND CLASSIFICATION OF NON-PERIODIC SIGNALS AND THE RESPECTIVE SYSTEM THAT IMPLEMENTS IT**

VERFAHREN ZUM ERKENNEN UND KLASSIFIZIEREN VON NICHTPERIODISCHEN SIGNALEN UND ENTSPRECHENDES SYSTEM, DAS DIESES VERFAHREN IMPLEMENTIERT

PROCÉDÉ DE DÉTECTION ET DE CLASSIFICATION DE SIGNAUX NON PÉRIODIQUES ET LE SYSTÈME CORRESPONDANT QUI LE MET EN OEUVRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2019 PT 2019116012**

(43) Date of publication of application:
**26.10.2022 Bulletin 2022/43**

(73) Proprietors:
• **Instituto Superior Técnico**
**1049-001 Lisboa (PT)**
• **INESC-ID Instituto de Engenharia de Sistemas e Computadores Investigação e Desenvolvimento de**
**Lisboa**
**1000-029 Lisboa (PT)**

(72) Inventors:
• **MIGUEL BÁRBARA COROAS PRISTA CAETANO, Diogo**
**1800-083 Lisboa (PT)**

• **GIBRAN RABUSKE KUNTZ, Taimur**
**1900-083 Lisboa (PT)**
• **GONÇALO NETO SILVA, João**
**2400-821 Leiria (PT)**
• **MANUEL DOS SANTOS RIBEIRO FERNANDES, Jorge**
**1900-428 Lisboa (PT)**
• **NUNO GOMES TAVARES, Gonçalo**
**2760-127 Caxias (PT)**

(74) Representative: **do Nascimento Gomes, Rui**
**J. Pereira da Cruz, S.A.**
**Rua Vítor Cordon, 10-A**
**1249-103 Lisboa (PT)**

(56) References cited:
**WO-A1-2019/067557**    **WO-A1-2020/030903**
**GB-A- 2 576 191**    **US-A1- 2017 333 903**
**US-A1- 2018 286 038**    **US-B2- 10 593 039**
**US-B2- 6 636 623**

**Description**

<u>INVENTION FIELD</u>

The present invention is within the technical area of Cytometry. In this context, the invention relates to particle detection methods, specifically, biological targets for the acquisition of biological information through flow cytometry. Specifically, the present invention relates to a classifier for biological target identification.

<u>BACKGROUND OF THE INVENTION</u>

The increase in the need for biological information at the cell unit level has led to the development of improved cytometry technologies. In particular, flow cytometry is a powerful method that has evolved a lot in the last few decades. Due to the complexity of the devices used in this technique, it is possible to identify an increasing number of cellular parameters. However, this increase in complexity and precision has also caused an increase in equipment production costs. On the other hand, with the development of microfluidic channel manufacturing techniques, it became possible to create smaller point-of-care devices. Generally, these devices are simpler and cheaper; however, producing a lower degree of precision with a reduced number of identified parameters. The search for increased precision while obtaining more information using these small devices has become a research field in itself.

A flow cytometer is typically comprised of the following elements: at least one channel, usually with a section in the order of square micrometers and of arbitrary length, where a biological sample suspended in flowing liquid medium is arranged; an excitation source, for instance, a laser, adapted to direct a light beam to the flowing liquid medium, or an applied magnetic field, whose purpose is to transfer a measurable character to the targets; a set of sensors used to detect labeling particles present in the biological sample; and a processing unit, configured to acquire, process and analyze the signals generated by the set of sensors in the sequence of detection events.

Magnetic flow cytometers (MFC) are an example of these platforms, which are often portable and of great potential (US6736978 B1). In this case, the most commonly used sensors use the quantum effects of Giant Magnetoresistance or Tunnel Magnetoresistance (Soares et al., 2019). Since there is very little or no magnetic content in biological samples, the use of MFC is dependent on the labeling of biological targets, with markers that exhibit magnetic behavior, so that, under certain conditions, they can produce a signal with a magnetic component, and thus, be detected by the set of sensors. Similarly, to optical flow cytometry (OFC), based on fluorophores, an MFC also uses biological probes such as antibodies, viruses, RNA, and others with an affinity for specific locations on targets. On MFC, these probes are functionalized with magnetic or paramagnetic elements - polarized by a magnetic excitation field - that allow their detection by magnetic sensors. For this purpose, the loaded biological sample is passed momentarily inside an MFC channel to be as close to the sensor(s) as possible. From the execution of the process described above, several challenges arise, both related to the detection of the signal produced by biological targets in a noisy environment and with the distinction between the signal produced by biological targets and the free magnetic markers - biological probes - used to detect them. In the state-of-the-art, the detection of signals produced by labeling particles is mostly performed through peak detection by applying thresholds and heuristic-based decision algorithms (Chícharo et al., 2018; Huang et al., 2017; Soares et al., 2019; US99934364 B1). The application of thresholds is very vulnerable to peaks caused by random noise and interference. In order to mitigate this vulnerability, most systems resort to the application of band-pass filtering, using a pass-band usually determined by limitations in the sampling system and not by the features of the signal, which makes it not optimal. In turn, the amplitude of the signal is determined by the proximity of the particle to be detected - (para)magnetic nanoparticles (MNP) in the case of MFC - to the set of sensors and by their respective magnetic content. In the case of OFC, the signal intensity is related to the intensity of the excitation applied to the fluorophores, characteristics of the fluorophores themselves, refraction and reflection indexes of the targets, and the internal content of the targets, among other factors.

Considering MFC as an illustrative example, the signal band is determined by the type and duration of the pulse, which depends on the target's speed in the flow, the MNPs size, and the sensor's size and shape. In the same flow, there may be MNPs moving at various speeds, which usually means that the pass-band is established considering the maximum band impulse. This approach is inefficient because it does not maximize the signal-to-noise ratio for pulses with energy in a smaller band.

In that sense, several strategies can be implemented to improve the signal-to-noise ratio, of which we highlight the approaches disclosed in Huang et al., 2017, and Chícharo et al., 2018. These approaches are divided into two categories: filter adaptation to the signal (Huang et al., 2017) or adaptation of the signal to the filter (Chícharo et al., 2018). However, despite being more efficient and personalized, the mentioned approaches do not allow a total adaptation to the single-impulse level and even to each individual experiment, being forced to maintain a certain level of generalization concerning the detected impulse in order not to lose possible candidates (e.g., broadband signals). At the same time, they are required to be very specific in the decision criteria. For example, to minimize the number of false positives caused by noise and interference, the applied amplitude decision threshold is usually about 4-5 times higher than the noise standard deviation, limiting detection to signals with a largely positive signal-to-noise ratio.

Another state-of-the-art methods for detecting non-periodic signals such as the one described in US2018/286038 uses an artificial neural network for the classification of biological target and suggest the use of a classifier as a stage of machine learning wherein an artificial neural network can be understood of several sequential layers: a biophysical feature space, a neural network hidden layer and a decision making layer. In the same context, document US6636623 suggest the use of two sequential classifiers for classifying a biological target in a flow cytometer. However, both documents are silent regarding a classification step comprised by sequential stages of machine learning, i.e., a detector classifier and an evaluator classifier.

Document WO2019/067557 teaches that a classifier should comprise producing a series of binary classifier to isolate a plurality of target cells derived from the plurality of first cells. Where a first classifier trained for isolation of malignant cells from other normal cells, and a second classifier for separating malignant subtypes. However, the first and the second classifiers do not respectively make the distinction between impulse verses non-impulse and between biological targets versus cluster of particles.

## TECHNICAL PROBLEMS SOLVED

State-of-the-art methods for detecting non-periodic signals, such as those produced by labeled particles associated with biological targets, within the scope of flow cytometry techniques for the acquisition of biological information, have shown to be insufficient in providing an optimized detection of the signal produced by biological targets in a noisy environment, their limits and specifications being defined according to extreme cases and not by individual pulses produced by the aforementioned biological targets. In this context, an individual impulse is generated by the interaction between labeled particles, which may or may not be associated with biological targets, and the sensors.

Especially for functions such as the proposal in Loureiro et al., 2011 and Chícharo et al., 2018, where MFC is used to count tumor cells, it is essential that the effectiveness and accuracy of the measurement are at 100% or close to it, instead of the lower percentages reported in the literature (Soares, et al., 2019). In fact, tumor cells are very rare and losing any of them in the count significantly impacts the disease's diagnosis or prognosis. Also, in the detection of pathogens used in chemical weapons, in the detection of multi-resistant and aggressive bacteria, or in the control of water quality, among others, the required precision cannot be guaranteed by the known methods due to their limitations: signal bands limited by hardware, complex and ineffective filtering steps, or exaggerated detection limits on measurement parameters in an effort to maximize the signal-to-noise ratio. In addition, the decision algorithms are generally simple and do not match the complexity that the filtering step requires.

In the present invention, a new approach is proposed for the detection and classification of the signal generated by labeled particles associated with biological targets, within the scope of flow cytometry techniques for biological information acquisition, which allows for an increased detection accuracy, overcoming the limitations identified in the state-of-the-art methods.

## INVENTION SUMMARY

The object of the present invention is a new method for the detection and classification of non-periodic signals and the respective system that implements it, within the scope of flow cytometry techniques for the acquisition of biological information, with the purpose of increasing the precision in the detection of patterns produced by labeled particles associated with biological targets.

The proposed objective is achieved by using a classifier based on machine learning techniques to execute a new detection methodology that combines the filtering and decision steps. In this way, more intelligence is put into the decision algorithm, and customization of the filter is achieved for each impulse generated by the interaction between labeling particles, associated with biological targets or not, and the detection sensors.

The classifier is applied to the detection of non-periodic signals, in particular, pulses of the type of the Gaussian family, with a bipolar or monopolar characteristic, allowing the distinction between a signal produced by a labeled particle and noise or interference, and thus provide an increase in the accuracy of the detection of biological targets in a biological sample.

## DESCRIPTION OF THE FIGURES

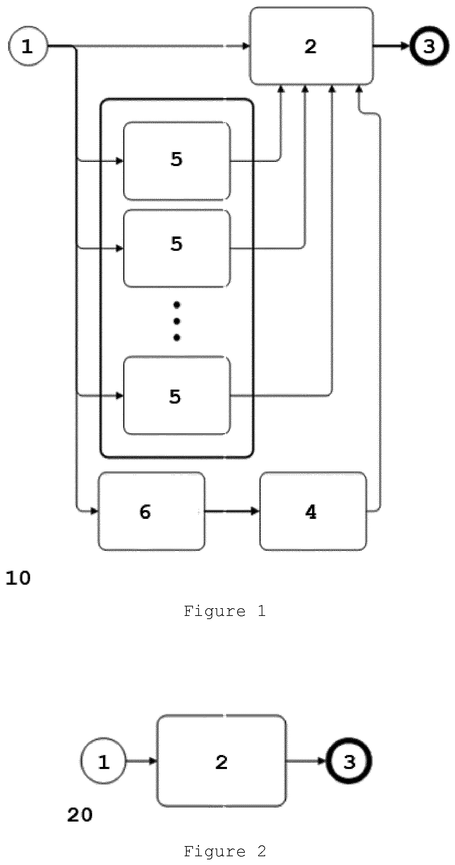

Figure 1 describes the architecture of a composed type classifier of the present invention, where the reference numeric signs mean:

(1) - input signal of the classifier;
(2) - ANN - artificial neural networks;
(3) - output of the classifier;
(4) - SVM - support vector machines;
(5) - regressor;
(6) - feature extraction algorithm;
(10) - composed classifier.

Figure 2 represents the architecture of a classifier of the independent type of the present invention, where the numeric reference signs mean:

(1) - input signal of the classifier;
(2) - ANN;
(3) - output of the classifier;
(20) - independent classifier.

Figure 3 represents the simulation of the interaction of a magnetic dipole with a sensor.

Figure 4 represents the filtered signal (41) resulting from the application of the flat band filter to a time sequence containing a bipolar pulse (42), in which the upper (43) and lower (44) thresholds used for the selection.

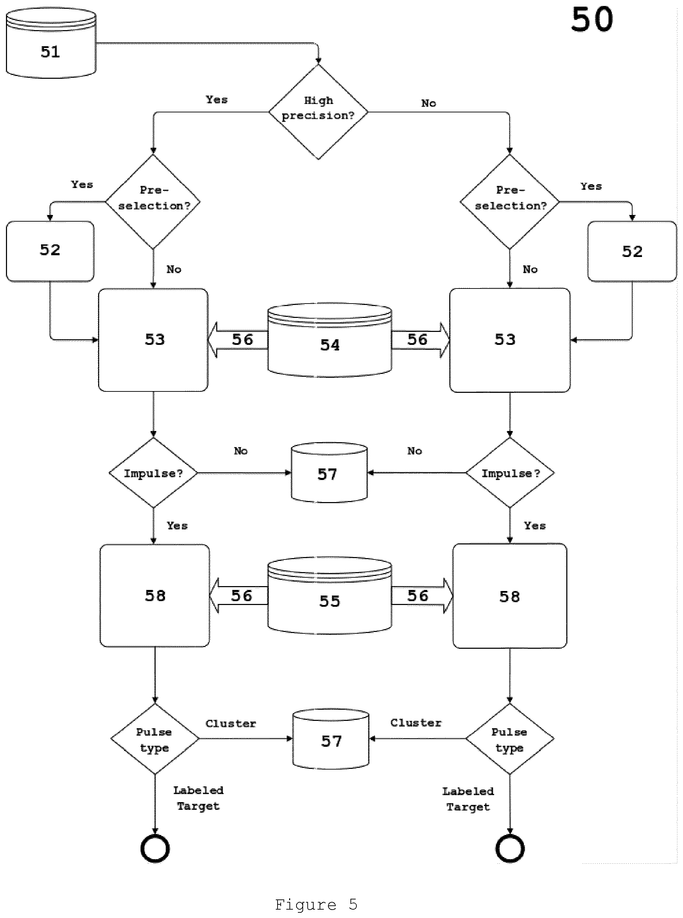

Figure 5 represents the flowchart illustrating the method of the invention (50), where the numerical reference signs mean:

(51) - time sequence;
(52) - pre-processing step;
(53) - detector classifier;
(54) - training set pulses vs. non-impulse";
(55) - training set labeled particle associated with biological target vs. clusters;
(56) - training stage;
(57) - elimination stage;
(58) - evaluator classifier;
(59) - detection event.

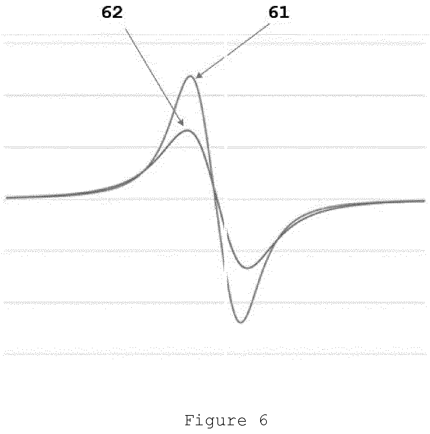

Figure 6 illustrates an example of the signal generated by the same number of particles when they are clustered in themselves or mark a biological target, where the reference number signs mean:

(61) - signal generated by cluster;
(62) - signal generated by a labeled particle associated with a biological target.

## DETAILED DESCRIPTION OF THE INVENTION

The present invention relates to a method (50) and the respective system that implements it, of signal processing to detect non-periodic signals, in particular, pulses of the Gaussian family, present in a sequence in time of limited duration (from now on only designated by time sequence) (51), and generated by sensors responsible for detecting signals emitted by biological targets, within the scope of flow cytometry techniques for the acquisition of biological information. Throughout the description, the terms signal and time sequence will be used to describe both the method (50) and the system of the invention. In this context, signal should mean the result of the processing carried out by the system, at each moment, of a limited set of data that constitutes a time sequence.

In particular, the present invention is related to a classifier based on machine learning, which provides an increased precision in the detection of labeled particles associated with biological targets.

The proposed classifier combines filtering and decision steps. First, general filtering is employed on the signal produced by the sensor, based on the maximum frequency band produced by the fastest events, as in the approaches already known. A liberal decision algorithm is then implemented, which does not need to minimize false positives, based on thresholds (43, 44) that may be closer to the noise standard deviation, estimated by analyzing the initial moments of the experiment where signals of interest are not yet present. Possible pulses are extracted and saved. Each possible pulse (42) is individually evaluated and filtered according to the band where it has the highest energy, resulting in a cleaner filtered signal (41). Subsequently, a decision classifier based on machine learning is applied, designated detector classifier (53), for pulse classification: positively classified pulses are evaluated by a subsequent machine learning classifier, called evaluator classifier (58). Both classifiers (53) and (58) can be of the independent (20) or composed (10) type. In the case of the composed (10) type classifier, by observing the nature of the signal, different features are extrapolated, depending on the cytometry technique being used. For example, in the case of an MFC, the features could be the speed of the target or the MNP, the angle of magnetization, the distance to the sensor, or the size of the target, whereas, in the case of an OFC, the features in question may be related to the linear speed of the target in the channel, the size of the target or the density of its content. Once the signal is characterized, the evaluator classifier (58), using this information and the time sequence itself, decides whether the pulses are originated from labeled particles associated with biological targets or from clusters of free particles.

The classifier allows detecting in an effective and intelligent way, non-periodic signals produced by the cytometer, which allows the identification of labeled particles associated with biological targets to be more accurate and for diagnoses based on this analysis to be more accurate, producing reduced false-positive rates compared to other detection methods used. This can be achieved because the intelligence of the classifier achieves two fundamental purposes for accurate detection: 1) to be able to identify a pattern in a noisy signal, said pattern normally belonging to the Gaussian family and 2) to distinguish the signal produced by a labeled particle associated with biological targets from the signal produced by a cluster of particles, not associated with biological targets.

The implementation of the classifier of the present invention may involve an initial stage of pre-selection of time sequences, extracted from raw information, through a threshold method. Candidates are filtered through a matched band-pass filter and subsequently evaluated by the classifier. Alternatively, a sliding window is applied to the raw data, which serves as a direct input to the classifier without any pre-selection.

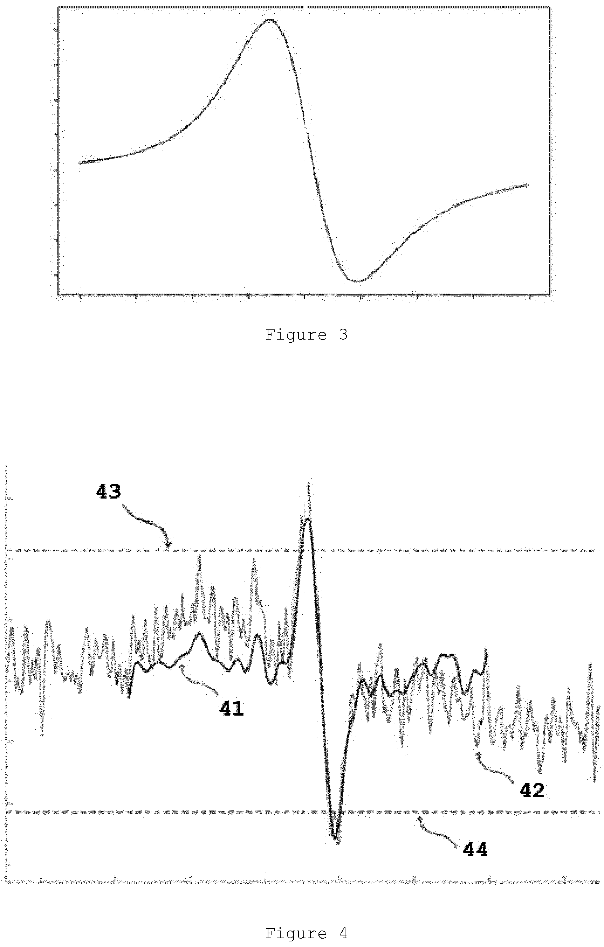

To implement the classifier, several regression methods are also implemented, which allow the extraction of additional information from each time sequence (51) that can serve as input to the classifier itself, such as: vertical distance from the target to the sensor (MFC), magnetization angle (MFC), and linear target speed on the channel (MFC or OFC), target size (MFC or OFC), etc. The targets or patterns to be found by the action of the classifier are Gaussian pulses, usually bipolar in the case of MFC or monopolar in the case of OFC, similar to those illustrated in Figure 3. One considers an impulse to be a time sequence, discrete or continuous, with only one cycle, of limited duration, during which there is a maximum or a minimum in the monopolar case, or a maximum and a minimum in the same absolute order of intensity in the bipolar case.

To accomplish this, the classifier's intelligence is conveyed by the use of two machine learning techniques - artificial neural networks (ANN) (2) and a support vector machine (SVM) (4) - that operate in a combined manner. In this context, the SVM (4) is used as an auxiliary classification method, serving its output as an input to the ANN (2). However, the SVM (4) can also be used independently to classify the pulses. To train (56) both the ANN (2) and the SVM (4), it is necessary to have a labeled training set (54, 55) in order to carry out supervised learning.

Training step - Simulation and Creation of training data

The training set (54, 55) and tests are based on digital signal acquisition simulations, which model the interaction between the labeled particles and the sensors (transducers) so that this results in a time sequence of samples with different numerical values. This type of simulations, when used to train (56) the machine learning algorithms, provide very precise results, being advantageous in relation to the use of real signals, namely due to the fact that the origin of the simulated signals is known and described, allowing the creation of the necessary labels for machine learning.

The interaction between targeted particles and the sensors is modeled according to the Cytometry technique in use. For example, in the case of an MFC, the pulses that the classifier intends to detect can be approximated by integrating a dipole field in the sensor's area.

In this sense, equation (1) describes the interaction of a magnetic dipole with a sensor of a specific area w×l (w - width, 1 - length) where x and y are the position of the dipole on the x and y axes, in relation to the center of the sensor and parallel to it, h is the position on the axis perpendicular to the sensor plane, M is the magnetization of the NPM, and Theta (θ) the magnetization angle of the MNP. This equation is then the basis for all simulations (MFC).

$$Hx_{avg} = \frac{\left( \int_{-\frac{l}{2}-y0}^{\frac{l}{2}-y0} \int_{-\frac{w}{2}-x0}^{\frac{w}{2}-x0} \frac{M}{4\pi} \frac{4\pi}{1000} \times \frac{3\cos\theta(x+h) + \sin\theta\, x^2}{(x^2+y^2+h^2)^{5/2}} - \frac{\sin\theta}{(x^2+y^2+h^2)^{3/2}} \mathrm{dxdy} \right)}{w \times l} \quad (1)$$

By fixing y, h, and θ, and sweeping x for a series of values, we can obtain the bipolar signal resulting from the particle interaction with the sensor. To obtain a training set (54, 55) for machine learning, for each scan of x, h and θ take a different value from a set of pre-selected values according to the process parameters on a cytometer, resulting in a bipolar pulse data-set. The height (h) is limited by the height of the channel, and θ is determined by the speed of rotation of the target moving in the fluid and the angle of the excitation magnetic field.

The set of signals resulting from digital simulations represents time sequences of samples without noise, which means that it does not resemble the real cases extracted from a cytometer. For the training set (54, 55) to contain cases that may be similar to reality, and therefore ensure that learning in training (56) translates to reality, it is necessary to carry out 3 expansion steps: sampling, adding noise, and generating a set of non-pulses.

In the sampling step, the continuous signal (the simulation is discrete, but any point can be simulated) is resampled with a different number of samples, thus adding the time/speed factor to the simulation and emulating the sampling process that takes place on the cytometer. For a computationally less demanding version, it is possible to make this sampling based on interpolated values from the original simulation instead of simulating all points.

In the noise addition step, noise with characteristics similar to that resulting from the cytometer is added to each of the time sequences. To increase the classifier's robustness, noise with different powers are added to each pulse. A repetition process is also necessary. It consists of adding to the same pulse several sets of random samples of noise with the same power, generating several versions of the noisy pulse. This is necessary because noise is a random phenomenon, and it is desirable that the classifier is insensitive to it and not to memorize a specific case of impulse with noise.

By creating A - different samples, N - noise powers, and R - repetitions, for each S - simulation, the resulting training set has $A \times N \times R \times S$ elements. This number can easily reach hundreds of thousands, starting from just a few dozen behavioral simulations.

The data-set with sampled pulses and noise of various powers is what will be presented to the classifier along with the corresponding label that represents how it should classify the pulses. However, for the classifier to learn to distinguish a desirable impulse - produced by a labeled particle - from everything else, it is also necessary to provide a similar amount of signals to disregard, called non-pulses. This database is created by generating noise and events similar to interference. Noise-only time sequences are created by generating sets of random samples with standard deviations equal to or similar to the noise added to the pulses. Interference is created by generating monopolar pulses, positive or negative, of different intensity and duration, in different positions along an observation window, to which noise is added.

Neural Network - Architecture

A preferable implementation structure of the classifier and regressor methods, using artificial neural networks, uses I entries, in which I results in the sum of the number of samples (N) in a time sequence to the number of features (F) extracted from that same time sequence, by a feature extraction algorithm (6), as well as the output of regressors (OR) (5) or of the SVM (OS) (4) that are simultaneously applied, I = N+(F+OR+OS). H hidden layers that are related to the complexity of the problem and size of the result-space to be analyzed can be used, and O outputs that depend on the number of classes ( $O = log_2 \lfloor number\ of\ classes \rfloor + 1$ , in the case of binary classifiers). For the purposes of the present invention, four classes were contemplated: impulse (class 1), non-impulse (class 2), impulse - labeled particle associated with biological target (class 1-1) and impulse - clusters (class 1-2). Although the classifier has two outputs, in fact, these are the result of two sequential phases: pulse detection (2 classes) and pulse evaluation (2 classes). As such, it was necessary to develop two classifiers that work in sequence. The first classifier, or detector classifier (53), is adapted to identify an impulse (class 1) and a non-impulse (class 2). The second classifier, or evaluator classifier (58), is adapted to distinguish, among the pulses detected by the first classifier, the type of impulse, that is, whether it is a labeled particle associated with a biological target (class 1-1) or a cluster (class 1-2).

For both the impulse detection task or the pulse evaluation task, independent (20) or composed (10) classifiers can be used. The type of classifier being determined by the available resources (time and hardware) and the difference in function is determined only by the training set (54, 55) to which they are submitted. For pulse detection, the detector classifier (53) is trained (56) with a training set (54) composed of examples of desirable pulses (class 1) and noise and interference, and/or 'non-pulses' (class 2). For impulse evaluation, the evaluator classifier (58) is trained with a training set (55), which comprises examples of labeled particle associated with biological target pulses (class 1-1) or cluster pulses (class 1 -2).

Independent (20) or composed (10) classifier

For class determination, the classifiers (53, 58) can be one of two types: independent (20) or composed (10) .

In an independent (20) classifier, the time sequences with N samples are used as I inputs, and the classification is based only on that information. In order to obtain the desired number of samples, for instance, a sliding window can be used, which goes through the experimental data gradually, sectioning the N samples that serve as input to the classifier (I = N).

The computational load associated with an independent (20) type classifier is lighter than the composed (10) type classifier, mainly because it does not require the extraction of features.

On the other hand, if a composed (10) type classifier is chosen, the inputs of the neural network I are the N samples of the time sequence, the extracted F features that can be simple mathematical operations on the values of the samples, as well as the outputs of the regressors (OR) or the SVM (OS), which leads to additional computation (I=N+F+OR+OS).

The regressors (5) used are created to estimate the variables used in the digital simulation, such as position in the channel and the linear speed, target size (knowing the sampling frequency), among others. Adding this information helps to increase the accuracy of the classification, but obviously requires more time and hardware resources. The regressors (5) are trained with a pulse data-set so that the values of the variables used in the simulation of each training case - number of labeled particles, distance to the sensor, speed, etc. - can be correctly estimated by the ANN (2), at the end of the training. Each regressor (5) can be dedicated to a simulation variable, or a single regressor (5) can have V outputs equal to the number of simulation variables to be inferred. The ANN (2) will be trained in order to minimize the error between the value of the variable that was used to simulate the impulse and the output of the ANN itself (2). After training, the bank of regressors (5), when subjected to an impulse, must produce as outputs all the values of the simulation variables that would produce an impulse equal to the one analyzed.

The feature extraction algorithm (6) used extracts information from the time sequence like, but not limited to: the maximum, the minimum, the number of samples between the maximum and the minimum, the width of the maximum or minimum for a given value of prominence, the maximum of the cumulative integral of all values, the width of the cumulative integral for a given prominence value, etc. Automatic methods of feature extraction, such as auto-encoders or principal component analysis, etc., can be used for this extraction.

SVM (4) is trained for analyzing the features extracted from the examples in the same database used for ANN (2) training.

Pre-Processing

The classifiers (53, 58) can be preceded by a pre-processing step (52) using thresholds and matched flat-band filtering with a linear phase delay, depending on the resources and time available.

This pre-processing (52) considers amplitude thresholds and a number of samples, and checks the respective ratio between maximum and minimum values (in the case of bipolar pulses). In this case, all time sequences, where at least within 'X' ($X \leq N$) samples, determined by the flow velocity, there is a maximum and/or a minimum greater in absolute value than a threshold - chosen as a multiple of the effective value of the noise, measured in the first samples of the raw signal -, and, in the case of bipolar pulses whose ratio 'R' between the maximum and the minimum, is within arbitrated limits, they are considered candidates for pulses.

For the detection using thresholds to be more accurate, a pseudo-differential measurement is used, in which the signal from a reference sensor is subtracted from the sensor signal to be measured. Since the sensors have different base resistance values, resulting from their manufacturing process - that can reach values of +/- 10% -, and also different biasing currents resulting from the mismatch between the biasing circuit (e.g. current sources), the signals have differences that must be compensated. To obtain this compensation, an estimation of this difference is performed to determine the multiplying factor that needs to be applied so that the subtraction of the two signals results in the lowest possible noise power possible. This factor is applied to the reference sensor's signal, and this is subtracted from the sensor signal to be measured. This subtraction removes or mitigates some of the interferences common to both sensors. It also allows the used amplitude-thresholds to be of a lower amplitude. The noise used for these steps is calculated based on the first test samples where no particles are present yet.

Subsequently, to facilitate the evaluation step, each candidate is filtered through a matched flat-band filter with a linear phase delay. This filter reduces the noise integration band as much as possible, so that the likely impulse is similar to the simulation. This is a digital filter whose bandwidth is adapted to contain a certain percentage of the energy contained in that time sequence. To determine the energy of the pulse, a spectral analysis is performed using the method of averages by segmented estimators of Welch or another similar method.

The proposed filter, in addition to improving the signal-to-noise ratio by reducing the integration band to a minimum, is more appropriate for this assessment task because the first classifier is trained with signals deformed only by noise, and not by unwanted effects of the channel (e.g., filter nonlinearities). With the use of this flat band filter, the impulse is deformed as little as possible, unlike what happens with the use of a common matched filter, which has the additional disadvantage of its interaction with a square pulse or similar, caused for instance by interference, resulting in an artifact very similar to the signal of a labeled particle associated with a biological target, which causes the appearance of patterns that would lead to the counting of false positives.

Although apparently more complex, pre-processing (52) requires, on average, less computational resources than required in the application of several matched filters, since only the first band-pass filter is applied to the entire signal, which has billions of time samples. The proposed matched band filters are only applied to a few hundred time sequences, depending on the number of events/particles in the sample and the chosen threshold, therefore containing a few thousand points.

First classifier (detector classifier)

The detector classifier (53) can be of the composed (10) or independent (20) type, depending on the level of performance required or the level of possible performance, taking into account the available time and hardware restrictions.

The detector classifier (53) used in this task must be trained (56) with a training set (54) composed of examples of desirable pulses (class 1) and noise and interference (class 2). After the detector classifier (53) is trained (56), the data can be classified. For this, a specific number of samples can be selected by a sliding window and inserted in the detector classifier (53), with the existence or not of the pre-processing step (52). After classification, the data is classified into pulses (class 1) and saved or non-pulses (class 2) and discarded.

Second classifier (evaluator classifier)

The evaluator classifier (58) can be of the composed (10) or independent (20) type, depending on the level of performance required or the level of possible performance, taking into account the available time and hardware restrictions.

After classification as non-impulse or impulse by the detector classifier (53), the time sequences identified as impulse are saved and submitted to a new classification round through the evaluator classifier (58), which was trained (56) to distinguish between clusters and labeled particle associated with biological target. This evaluator classifier (58) is trained (56) with a training set (55) composed, for instance, of pulses generated either by labeled particles associated with biological targets (class 1-1) or by clusters of particles (class 1-2). After training (56) the evaluator classifier (58), the data can be evaluated. For this, they are selected by the sliding window and inserted in the evaluator classifier (58), going or not through the pre-processing step (52), ending in a detection event (59), each data being classified as a labeled particle associated with a biological target (class 1-1) and saved or classified as a cluster (class 1-2) and discarded in the elimination step (57).

Cluster distinction

The purpose of the evaluator classifier (58) is to distinguish between pulses generated by labeled particles associated with biological targets and pulses generated only by clusters. Figure 6 shows an example of the signal generated by the same number of particles, when they are clustered together, signal generated by cluster (61), or when they label a biological target, signal generated by a labeled particle associated with a biological target (62). It is possible to see that the waves share the duration, but the amplitude is smaller in the case of the labeled particle associated with a biological target. The decrease in amplitude is due to the fact that, in the biological target/labeled particles complex, more particles are at a greater distance from the sensor, particularly on the axis perpendicular to the sensor plane when compared to a cluster of particles.

In this way, the evaluator classifier (58) is trained (56) with a simulation training set (55) that contains simulated signals of labeled particles associated with biological targets (class 1-1) and clusters (class 1-2) and with respective tags. To do this, in the case of the independent classifier (20), the time sequence that represents the impulse is used as input, and in the case of the composed classifier (10), the input is complemented with the features of the target/particle extracted by the regressors , such as: distance to the sensor, speed, target size, etc., in conjunction with the additional signal features mentioned above: the maximum, the minimum, the number of samples between the maximum and the minimum, the width of the maximum or minimum for a given value of prominence, the maximum of the cumulative integral of all values, the width of the cumulative integral for a given prominence value, etc. and also the output of the SVM.

The specified inputs can all be used simultaneously, independently, or combined as is most convenient for the application. Unlike the detector classifier (53), which distinguishes pulses from noise, in which one of the training classes is composed of pulses and the other of noise and interference, for the evaluator classifier (58), the training set is only composed of pulses from different origins, which allows it to specialize only in this distinction.

Classic signal processing

The developed classifier can be applied in conjunction with classic signal processing methods, such as hardware or software filters, along with heuristics. However, the intelligence that the machine learning algorithms provide makes it possible to apply the classifier directly to the raw signal, at the output of the cytometer, without previous filtering or selection steps.

# EP 4 080 191 B1

## REFERENCES

Chícharo, A., Martins, M., Barnsley, LC, Taouallah, A., & Fernandes, J. (2018). Enhanced magnetic microcytometer with 3D flow focusing for cell enumeration. doi: https: //doi.org/10.1039/C8LC00486B.

Soares, R., Martins, VC, Macedo, R., Cardoso, FA, Martins, SA, Caetano, DM, Henriques, P., Silvério, V., Cardoso, S., Freitas, PP (2019). Go with the flow: advances and trends in magnetic flow cytometry. Analytical and Bioanalytical Chemistry, 411 (9), 1839-1862.

Huang, C., X, Z., Ying, D., & Hall, D. (2017). A GMR-based magnetic flow cytometer using matched filtering. IEEE Sensors, p. 1-3.

Loureiro, J., Andrade, PZ, Cardoso, S, Silva, CL, Cabral, JM, & Freitas, PP (2011). Magnetoresistive chip cytometer. Lab on a Chip, 2255-2261.

## Claims

1. Method for detection and classification of non-periodic signals emitted by biological targets within the scope of flow cytometry techniques, comprising the following steps:

   - acquisition of at least one-time sequence (51) generated by a detection sensor;
   - filtering the signal produced by the sensor, based on its maximum band;
   - extraction and storage of impulse candidates by implementing a liberal decision algorithm, based on noise standard deviation threshold values;
   - individual filtering of each impulse candidate, according to its highest energy band;

   the method being **characterized by** comprising the steps of:

   - classification, comprising two sequential stages of machine learning; a first stage, detector classifier (53), adapted to classify an impulse candidate as impulse or non-impulse; a second stage, evaluator classifier (58), adapted to classify the pulses, identified in the first stage, as a labeled particle associated with a biological target or as a cluster of particles; and
   - counting the number of pulses classified as labeled particles associated with a biological target in the detection event (59).

2. Method according to claim 1, wherein the classification step implements regression methods and feature extraction algorithms (6) from the time sequence (51) that serve as input to the two stages of machine learning, classifiers (53, 58).

3. Method according to claim 2, wherein extrapolated features are obtained from feature extraction algorithms (6) implemented in the classification step; said extrapolated features being dependent on the Cytometry technique to be performed.

4. Method according to claim 3, wherein the extrapolated features are:

   the speed of the target or the magnetic nanoparticle, the angle of magnetization, the distance to the sensor or the size of the target, in case the Cytometry technique to be performed is Magnetic Flow Cytometry - MFC; or
   the target's speed in the channel, the size of the target or the density of the target content, in case the Cytometry technique to be performed is the Optical Flow Cytometry - OFC.

5. Method according to any of the preceding claims, wherein the two stages of machine learning, classifiers (53, 58), to be performed in the classification step, are fed from training sets (54, 55), in which;

   the first machine learning stage, detector classifier (53) is trained (56) with a training set (54) composed of examples of pulses, examples of noise and interference, and examples of non-pulses;

9

and the second stage of machine learning, the evaluator classifier (58) is trained (56) with a training set (55) composed of examples of labeled particles associated with biological target pulses and examples of cluster pulses.

6. Method according to claim 5, wherein the training set (54) that feeds the first machine learning stage, detector classifier (53), is adapted to identify pulses of the Gaussian family; said Gaussian pulses being bipolar in the case of MFC or monopolar in the case of OFC.

7. Method according to claim 5 or 6, wherein the training set (54) that feeds the first machine learning stage, detector classifier (53), is generated from 3 expansion steps:

   - generation of a time sequence of samples resulting from digital simulations adapted to model the interaction between a labeled particle and a detection sensor; said time sequences being subsequently sampled;
   - addition to each of the sampled time sequences, noise with characteristics similar to the noise produced in a cytometer;
   - generation of a set of non-pulses, through the generation of noise and events similar to interference; and wherein,

   the sampling of time sequences resulting from digital simulations involves:

   - a resampling process with a different number of samples; or
   - interpolation of the digital simulation.

8. Method according to claims 7, wherein the addition of noise involves:

   - adding noise of different powers to each pulse; and
   - the addition of several sets of random noise samples with the same power to the same pulse.

9. Method according to claims 7 or 8, wherein

   the noise, in the non-impulse generation step, is generated from random samples with standard deviations equal to or similar to the noise added to the pulses in the noise addition step; and
   the interference is generated through monopolar pulses of different intensities and durations.

10. Method according to any of the preceding claims, wherein the second stage of machine learning, evaluator classifier (58), is configured to distinguish an impulse from a labeled particle associated with a biological target, from an impulse generated from a cluster of particles; that said distinction including the steps of:

    - extrapolation of features from the time sequence under analysis;
    - execution of an evaluation algorithm that has as input the time sequence (51) of an impulse marked in the first machine learning stage as an impulse and the features extracted from the time sequence (51).

11. Method according to any one of the preceding claims, wherein it includes an additional pre-processing step (52) before the classifiers (53, 58); the said pre-processing step (52) involves the step of detecting an impulse candidate based on amplitude thresholds and number of samples; said threshold detection using pseudo-differential measurement where the time sequence generated by a detection sensor is subtracted from the time sequence generated by a reference sensor.

12. Method according to any one of the preceding claims, wherein the introduction between the first machine learning stage, detector classifier (53), and the second machine learning stage, evaluator classifier (58), of a new pre-processing step (52) where each impulse identified in the first stage, detector classifier (53), is filtered through a flat-band matched filter and linear phase delay; and wherein, the filter is digital and has a pass-band adapted to contain a specific percentage of the energy contained in the time sequence; the percentage of energy being determined through spectral analysis.

13. System for the detection and classification of non-periodic signals comprising:

    - A detector classifier (53) and an evaluator classifier (58) adapted to execute machine learning algorithms; the system being **characterized in that**
- Each classifier (53, 58) being formed by at least one artificial neural network (2) and being adapted to perform the method of claims 1 to 12.

14. System according to claim 13, wherein each classifier (53, 58) additionally comprises a support vector machine (4), wherein the output of said machine (4) is an input to the artificial neural network (2).

15. System according to claim 14, wherein each classifier (53, 58) additionally comprises at least one regressor (5), wherein

at least one regressor (5) is adapted to estimate the variables used in the digital simulation, such as: position in the channel, speed or target size; and
the output of a regressor (5) is an input to the artificial neural network (2); and wherein,
each classifier (53, 58) comprises an artificial neural network (2) composed of:

- *I* entries, resulting from the sum of:

- a number of samples, N, in a time sequence (51) to be processed;
- a number of features, F, extracted from said time sequence (51) and resulting from a feature extraction algorithm (6) executable by the classifier (53, 58);
- a number of regressor (5) outputs, OR; and
- an output of the support vector machine (4), *OS;*

- *O* number of outputs, wherein $O = log_2 \lfloor number\ of\ classes \rfloor + 1$ .

**Patentansprüche**

1. Verfahren zur Detektion und Klassifizierung von nichtperiodischen Signalen, die von biologischen Zielen im Rahmen von Durchflusszytometrie-Techniken ausgesendet werden, umfassend die folgenden Schritte:

- Erfassung von mindestens einer Zeitsequenz (51), die von einem Detektionssensor erzeugt wird;
- Filterung des vom Sensor erzeugten Signals auf der Grundlage seiner maximalen Bandbreite;
- Extraktion und Speicherung von Impulskandidaten durch Implementierung eines Algorithmus zur freien Entscheidung auf der Grundlage von Schwellenwerten für die Rauschstandardabweichung;
- individuelle Filterung jedes Impulskandidaten auf der Grundlage seines höchsten Energiebandes;

das Verfahren ist **dadurch gekennzeichnet, dass** es die folgenden Schritte umfasst:

- Klassifizierung, die zwei aufeinanderfolgende Stufen des maschinellen Lernens umfasst; eine erste Stufe, Detektor-Klassifikator (53), der angepasst ist, um einen Impulskandidaten als Impuls oder Nicht-Impuls zu klassifizieren; eine zweite Stufe, Auswerter-Klassifikator (58), der angepasst ist, um die Impulse, die in der ersten Stufe identifiziert wurden, als ein markiertes Partikel, das mit einem biologischen Ziel verbunden ist, oder als ein Cluster von Partikeln zu klassifizieren; und
- Zählen der Anzahl von Impulsen, die als markierte Partikel, die mit einem biologischen Ziel assoziiert sind, klassifiziert wurden, in dem Detektionsereignis (59).

2. Verfahren nach Anspruch 1, wobei der Klassifizierungsschritt Regressionsmethoden und Merkmalsextraktionsalgorithmen (6) aus der Zeitsequenz (51) implementiert, die als Eingabe für die beiden Stufen des maschinellen Lernens, Klassifikatoren (53, 58), dienen.

3. Verfahren nach Anspruch 2, wobei extrapolierte Merkmale aus Merkmalsextraktionsalgorithmen (6) erhalten werden, die im Klassifizierungsschritt implementiert werden, wobei die extrapolierten Merkmale von der durchzuführenden Zytometrie-Technik abhängig sind.

4. Verfahren nach Anspruch 3, wobei die extrapolierten Merkmale sind:

die Geschwindigkeit des Ziels oder des magnetischen Nanopartikels, der Magnetisierungswinkel, die Entfernung zum Sensor oder die Größe des Ziels, falls die durchzuführende Zytometrie-Technik die magnetische Durchflusszytometrie (Magnetic Flow Citometry - MFC) ist; oder

die Geschwindigkeit des Ziels im Kanal, die Größe des Ziels oder die Dichte des Zielinhalts, falls die durchzuführende Zytometrie-Technik die optische Durchflusszytometrie (Optical Flow Citometry - OFC) ist.

5. Verfahren nach einem der vorhergehenden Ansprüche, wobei die beiden Stufen des maschinellen Lernens, Klassifikatoren (53, 58), die im Klassifizierungsschritt durchgeführt werden sollen, aus Trainingssätzen (54, 55) gespeist werden, in denen;

   die erste Stufe des maschinellen Lernens, Detektor-Klassifikator (53), mit einem Trainingssatz (54) trainiert (56) wird, der aus Beispielen von Impulsen, Beispielen von Rauschen und Interferenzen und Beispielen von Nicht-Impulsen besteht;
   und der zweiten Stufe des maschinellen Lernens, Auswerter-Klassifikator (58), mit einem Trainingssatz (55) trainiert (56) wird, der aus Beispielen von markierten Partikeln, die mit biologischen Zielimpulsen assoziiert sind, und aus Beispielen von Clusterimpulsen besteht.

6. Verfahren nach Anspruch 5, wobei der Trainingssatz (54), die die erste Stufe des maschinellen Lernens, Detektor-Klassifikator (53), speist, so angepasst ist, dass sie Impulse der Gauß-Familie identifiziert, wobei die Gauß-Impulse im Fall von MFC bipolar oder im Fall von OFC monopolar sind.

7. Verfahren nach Anspruch 5 oder 6, wobei der Trainingssatz (54), die die erste Stufe des maschinellen Lernens, Detektor-Klassifikator (53), speist, aus drei Expansionsschritten erzeugt wird:

   - Erzeugung einer Zeitsequenz von Proben, die sich aus digitalen Simulationen ergeben, die geeignet sind, die Wechselwirkung zwischen einem markierten Partikel und einem Detektionssensor zu modellieren; wobei die Zeitsequenzen anschließend abgetastet werden;
   - Hinzufügen von Rauschen zu jeder der abgetasteten Zeitsequenzen, das ähnliche Eigenschaften wie das in einem Zytometer erzeugte Rauschen aufweist;
   - Erzeugung eines Satzes von Nicht-Impulsen durch die Erzeugung von Rauschen und interferenzähnlichen Ereignissen;
   und wobei,

   die Abtastung von Zeitsequenzen, die sich aus digitalen Simulationen ergeben, Folgendes umfasst:

   - eine erneute Abtastung mit einer anderen Anzahl von Proben; oder
   - eine Interpolation der digitalen Simulation.

8. Verfahren nach Anspruch 7, wobei das Hinzufügen von Rauschen Folgendes umfasst:

   - das Hinzufügen von Rauschen unterschiedlicher Leistung zu jedem Impuls; und
   - das Hinzufügen mehrerer Sätze von zufälligen Rauschproben mit der gleichen Leistung zu demselben Impuls.

9. Verfahren nach Anspruch 7 oder 8, wobei

   das Rauschen in dem Nicht-Impuls-Erzeugungsschritt aus Zufallsproben mit Standardabweichungen erzeugt wird, die gleich oder ähnlich dem Rauschen sind, das den Impulsen in dem Rauschhinzufügungsschritt hinzugefügt wird; und
   die Interferenz durch monopolare Impulse mit unterschiedlicher Intensität und Dauer erzeugt wird.

10. Verfahren nach einem der vorhergehenden Ansprüche, wobei die zweite Stufe des maschinellen Lernens, Auswerter-Klassifikator (58), so konfiguriert ist, dass er einen Impuls von einem markierten Partikel, das mit einem biologischen Ziel assoziiert ist, von einem Impuls unterscheidet, der von einem Cluster von Partikeln erzeugt wird; wobei die Unterscheidung die folgenden Schritte umfasst:

   - Extrapolation von Merkmalen aus der zu analysierenden Zeitsequenz;
   - Ausführung eines Auswertealgorithmus, dessen Eingabe die Zeitsequenz (51) eines Impulses ist, der in der ersten Stufe des maschinellen Lernens als Impuls markiert wurde, sowie die aus der Zeitsequenz (51) extrahierten Merkmale.

11. Verfahren nach einem der vorhergehenden Ansprüche, wobei es einen zusätzlichen Vorverarbeitungsschritt (52) vor den Klassifikatoren (53, 58) umfasst; wobei der Vorverarbeitungsschritt (52) den Schritt der Detektion eines Impulskandidaten auf der Grundlage von Amplitudenschwellen und der Anzahl von Proben umfasst; wobei die Schwellendetektion eine pseudodifferentielle Messung verwendet, bei der die von einem Detektionssensor erzeugte Zeitsequenz von der von einem Referenzsensor erzeugten Zeitsequenz subtrahiert wird.

12. Verfahren nach einem der vorhergehenden Ansprüche, wobei zwischen der ersten Stufe des maschinellen Lernens, Detektor-Klassifikator (53), und der zweiten Stufe des maschinellen Lernens, Auswerter-Klassifikator (58), ein neuer Vorverarbeitungsschritt (52) eingeführt wird, bei dem jeder in der ersten Stufe, Detektor-Klassifikator (53), identifizierte Impuls durch ein angepasstes Flachbandfilter und eine lineare Phasenverzögerung gefiltert wird; und wobei, das Filter digital ist und ein Durchlassband hat, das so angepasst ist, dass es einen bestimmten Prozentsatz der in der Zeitsequenz enthaltenen Energie enthält; wobei der Prozentsatz der Energie durch Spektralanalyse bestimmt wird.

13. System zur Detektion und Klassifizierung von nichtperiodischen Signalen, das Folgendes umfasst:

   - Mindestens einen Klassifikator, der geeignet ist, Algorithmen des maschinellen Lernens auszuführen; wobei der Klassifikator aus zwei Klassifikatoren (53, 58) besteht; wobei das System **dadurch gekennzeichnet ist, dass**
   - jeder Klassifikator (53, 58) durch mindestens ein künstliches neuronales Netz (2) gebildet wird und geeignet ist, das Verfahren der Ansprüche 1 bis 12 auszuführen.

14. System nach Anspruch 13, wobei jeder Klassifikator (53, 58) zusätzlich eine Support-Vektor-Maschine (4) umfasst, wobei die Ausgabe der Maschine (4) eine Eingabe für das künstliche neuronale Netz (2) ist.

15. System nach Anspruch 14, wobei jeder Klassifikator (53, 58) zusätzlich mindestens einen Regressor (5) umfasst, wobei

   mindestens ein Regressor (5) angepasst ist, um die in der digitalen Simulation verwendeten Variablen zu schätzen, wie z.B.: Position im Kanal, Geschwindigkeit oder Zielgröße; und
   die Ausgabe eines Regressors (5) eine Eingabe für das künstliche neuronale Netz (2) ist; und wobei,
   jeder Klassifikator (53, 58) umfasst ein künstliches neuronales Netz (2), bestehend aus:

      - *I* Einträgen, die sich aus der Summe von Folgendem ergeben:

         - einer Anzahl von Proben, N, in einer zu verarbeitenden Zeitsequenz (51);
         - einer Anzahl von Merkmalen, F, die aus der genannten Zeitsequenz (51) extrahiert werden und sich aus einem Merkmalsextraktionsalgorithmus (6) ergeben, der von dem Klassifikator (53, 58) ausgeführt werden kann;
         - einer Anzahl von Regressor (5)-Ausgaben, *OR;* und
         - einer Ausgabe der Support-Vektor-Maschine (4), *OS;*

      - O Anzahl der Ausgaben, wobei $O = log_2 \lfloor Anzahl\ der\ Klassen \rfloor + 1$.

**Revendications**

1. Procédé de détection et de classification de signaux non périodiques émis par des cibles biologiques dans le cadre de techniques de cytométrie en flux, comprenant les étapes suivantes :

   - acquisition d'au moins une séquence (51) unique générée par un capteur de détection ;
   - filtrage du signal produit par le capteur, sur la base de sa bande maximale ;
   - extraction et stockage d'impulsions candidates en mettant en œuvre un algorithme de décision libéral, sur la base des valeurs seuils d'écart type du bruit ;
   - filtrage individuel de chaque impulsion candidate, selon sa bande d'énergie la plus élevée ;

   le procédé étant **caractérisé en ce qu'**il comprend les étapes suivantes :

- classification, comprenant deux étapes séquentielles d'apprentissage automatique ; une première étape, le classificateur détecteur (53), adaptée pour classer une impulsion candidate comme impulsion ou non-impulsion ; une deuxième étape, le classificateur évaluateur (58), adaptée pour classer les impulsions, identifiées dans la première étape, comme une particule étiquetée associée à une cible biologique ou comme un groupe de particules ; et
- compter le nombre d'impulsions classées comme particules étiquetées associées à une cible biologique dans l'événement de détection (59).

2. Procédé selon la revendication 1, dans lequel l'étape de classification met en œuvre des méthodes de régression et des algorithmes d'extraction de caractéristiques (6) à partir de la séquence temporelle (51) qui servent d'entrée aux deux étapes d'apprentissage automatique, les classificateurs (53, 58).

3. Procédé selon la revendication 2, dans lequel les caractéristiques extrapolées sont obtenues à partir d'algorithmes d'extraction de caractéristiques (6) mis en œuvre dans l'étape de classification ; lesdites caractéristiques extrapolées dépendant de la technique de cytométrie à être exécutée.

4. Procédé selon la revendication 3, dans lequel les caractéristiques extrapolées sont :

la vitesse de la cible ou de la nanoparticule magnétique, l'angle d'aimantation, la distance par rapport au capteur ou la taille de la cible, dans le cas où la technique de cytométrie à exécuter est la cytométrie en flux magnétique (MFC) ; ou
la vitesse de la cible dans le canal, la taille de la cible ou la densité du contenu de la cible, dans le cas où la technique de cytométrie à exécuter est la cytométrie en flux optique (OFC).

5. Procédé selon l'une quelconque des revendications précédentes, dans lequel les deux étapes d'apprentissage automatique, les classificateurs (53, 58), à exécuter dans l'étape de classification sont alimentées à partir d'ensembles d'apprentissage (54, 55), dans lesquels :

la première étape d'apprentissage automatique, le classificateur détecteur (53), est entraînée (56) avec un ensemble d'apprentissage (54) composé d'exemples d'impulsions, d'exemples de bruit et d'interférence, et d'exemples de non-impulsions ;
et la deuxième étape d'apprentissage automatique, le classificateur évaluateur (58), est entraînée (56) avec un ensemble d'apprentissage (55) composé d'exemples de particules étiquetées associées à des impulsions de cibles biologiques et d'exemples d'impulsions d'agrégats (de particules).

6. Procédé selon la revendication 5, dans lequel l'ensemble d'apprentissage (54) qui alimente la première étape d'apprentissage automatique, le classificateur détecteur (53), est adapté pour identifier des impulsions de la famille gaussienne ; lesdites impulsions gaussiennes étant bipolaires dans le cas du MFC ou monopolaires dans le cas de l'OFC.

7. Procédé selon la revendication 5 ou 6, dans lequel l'ensemble d'apprentissage (54) qui alimente la première étape d'apprentissage automatique, le classificateur détecteur (53), est généré à partir de 3 étapes d'expansion :

- génération d'une séquence temporelle d'échantillons résultant de simulations numériques adaptées pour modéliser l'interaction entre une particule étiquetée et un capteur de détection ; lesdites séquences temporelles étant ensuite échantillonnées ;
- ajout à chacune des séquences temporelles échantillonnées d'un bruit présentant des caractéristiques similaires au bruit produit dans un cytomètre ;
- génération d'un ensemble de non-impulsions, au moyen de la génération de bruit et d'événements similaires à des interférences ;
et dans lequel,

l'échantillonnage des séquences temporelles résultant de simulations numériques implique :

- un processus de rééchantillonnage avec un nombre différent d'échantillons ; ou
- une interpolation de la simulation numérique.

8. Procédé selon la revendication 7, dans lequel l'ajout de bruit implique :

- l'ajout de bruit de différentes puissances à chaque impulsion ; et
- l'ajout de plusieurs ensembles d'échantillons de bruit aléatoire de même puissance à la même impulsion.

9. Procédé selon les revendications 7 ou 8, dans lequel

le bruit, dans l'étape de génération de non-impulsions, est généré à partir d'échantillons aléatoires avec des écarts types égaux ou similaires au bruit ajouté aux impulsions dans l'étape d'ajout de bruit ; et
l'interférence est générée au moyen d'impulsions monopolaires de différentes intensités et durées.

10. Procédé selon l'une quelconque des revendications précédentes, dans lequel la deuxième étape d'apprentissage automatique, le classificateur évaluateur (58), est configurée pour distinguer une impulsion provenant d'une particule étiquetée associée à une cible biologique d'une impulsion générée à partir d'un agrégat de particules ; ladite distinction comprenant les étapes suivantes :

- extrapolation des caractéristiques à partir de la séquence temporelle analysée ;
- exécution d'un algorithme d'évaluation qui a comme entrée la séquence temporelle (51) d'une impulsion marquée dans la première étape d'apprentissage automatique comme une impulsion et les caractéristiques extraites de la séquence temporelle (51).

11. Procédé selon l'une quelconque des revendications précédentes, lequel comprend une étape de prétraitement (52) supplémentaire avant les classificateurs (53, 58) ; ladite étape de prétraitement (52) implique l'étape de détection d'une impulsion candidate sur la base de seuils d'amplitude et d'un nombre d'échantillons ; ladite détection de seuils utilisant une mesure pseudo-différentielle où la séquence temporelle générée par un capteur de détection est soustraite de la séquence temporelle générée par un capteur de référence.

12. Procédé selon l'une quelconque des revendications précédentes, dans lequel l'introduction entre la première étape d'apprentissage automatique, le classificateur détecteur (53), et la deuxième étape d'apprentissage automatique, le classificateur évaluateur (58), d'une nouvelle étape de prétraitement (52) où chaque impulsion identifiée dans la première étape, le classificateur détecteur (53), est filtrée au moyen d'un filtre adapté à bande plate et un retard de phase linéaire ; et dans lequel,
le filtre est numérique et a une bande passante adaptée pour contenir un pourcentage spécifique de l'énergie contenue dans la séquence temporelle ; le pourcentage d'énergie étant déterminé par analyse spectrale.

13. Système pour la détection et la classification de signaux non périodiques comprenant :

- au moins un classificateur adapté pour exécuter des algorithmes d'apprentissage automatique ; ledit classificateur étant composé de deux classificateurs (53, 58) ;

le système étant **caractérisé en ce que**

- chaque classificateur (53, 58) est formé d'au moins un réseau neuronal artificiel (2) et est adapté pour mettre en œuvre le procédé selon les revendications 1 à 12.

14. Système selon la revendication 13, dans lequel chaque classificateur (53, 58) comprend en outre une machine à vecteurs de support (4), dans lequel la sortie de ladite machine (4) est une entrée vers le réseau neuronal artificiel (2).

15. Système selon la revendication 14, dans lequel chaque classificateur (53, 58) comprend en outre au moins un régresseur (5), dans lequel

au moins un régresseur (5) est adapté pour estimer les variables utilisées dans la simulation numérique, telles que : la position dans le canal, la vitesse ou la taille de la cible ; et
la sortie d'un régresseur (5) est une entrée vers le réseau neuronal artificiel (2) ; et dans lequel,
chaque classificateur (53, 58) comprend un réseau neuronal artificiel (2) composé de :

- $I$ entrées, résultant de la somme de :

- un nombre d'échantillons, N, dans une séquence temporelle (51) à traiter ;
- un nombre de caractéristiques, F, extraites de ladite séquence temporelle (51) et résultant d'un algorithme d'extraction de caractéristiques (6) exécutable par le classificateur (53, 58) ;
- un nombre de sorties de régresseur (5), OR ; et
- une sortie de la machine à vecteurs de support (4), OS ;

- O nombre de sorties, où $O = log_2 \lfloor nombre\ de\ classes \rfloor + 1$.

EP 4 080 191 B1

Figure 1

Figure 2

Figure 3

Figure 4

Figure 5

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6736978 B1
- US 99934364 B1
- US 2018286038 A
- US 6636623 B
- WO 2019067557 A

**Non-patent literature cited in the description**

- **CHÍCHARO, A.** ; **MARTINS, M.** ; **BARNSLEY, LC** ; **TAOUALLAH, A.** ; **FERNANDES, J.** *Enhanced magnetic microcytometer with 3D flow focusing for cell enumeration*, 2018
- **SOARES, R.** ; **MARTINS, VC** ; **MACEDO, R.** ; **CARDOSO, FA** ; **MARTINS, SA** ; **CAETANO, DM** ; **HENRIQUES, P.** ; **SILVÉRIO, V.** ; **CARDOSO, S.** ; **FREITAS, PP**. Go with the flow: advances and trends in magnetic flow cytometry. *Analytical and Bioanalytical Chemistry*, 2019, vol. 411 (9), 1839-1862
- **HUANG, C.** ; **X, Z.** ; **YING, D.** ; **HALL, D.** A GMR-based magnetic flow cytometer using matched filtering. *IEEE Sensors*, 2017, 1-3
- **LOUREIRO, J.** ; **ANDRADE, PZ** ; **CARDOSO, S** ; **SILVA, CL** ; **CABRAL, JM** ; **FREITAS, PP**. Magnetoresistive chip cytometer. *Lab on a Chip*, 2011, 2255-2261